# FASCICULE DE BREVET EUROPEEN

(11) **EP 0 889 863 B1**
(45) Date de publication et mention de la délivrance du brevet: **07.05.2003**
(21) Numéro de dépôt: 97951051.8
(22) Date de dépôt: 30.12.1997
(51) Int. Cl.: C04B 41/00

(54) **MATERIAUX CARBONES MODIFIES EN SURFACE**
OBERFLÄCHENMODIFIZIERTE KOHLENSTOFFMATERIALIEN
SURFACE MODIFIED CARBONATE MATERIALS

(30) Priorité: 30.12.1996 CA 2194127; 05.03.1997 CA 2199231
(43) Date de publication de la demande: 13.01.1999
(73) Titulaire: HYDRO-QUEBEC, Montréal Québec H2Z 1A4 (CA); CENTRE NATIONAL DE LA RECHERCHE SCIENTIFIQUE (CNRS), 75016 Paris (FR)
(72) Inventeur: MICHOT, Christophe, F-38000 Grenoble (FR); ARMAND, Michel, Montréal, Québec H3T 1NZ (CA); GAUTHIER, Michel, La Prairie, Québec J5R 1E6 (CA); CHOQUETTE, Yves, Sainte-Julie, Québec J3E 1PE (CA)
(74) Mandataire: Sueur, Yvette
(86) Numéro de dépôt international: CA9701008
(87) Numéro de publication internationale: WO98029358

(56) Documents cités:
- EP-A- 0 289 624
- EP-A- 0 475 075
- EP-A- 0 635 380
- EP-A- 0 753 548
- US-A- 4 558 087
- US-A- 4 714 569
- US-A- 4 835 074
- DATABASE WPI Week 8930 Derwent Publications Ltd., London, GB; AN 89-215747 XP002058570 & JP 01 152 165 A (NIPPON SHOKUBAI) , 14 juin 1989
- DATABASE WPI Section Ch, Derwent Publications Ltd., London, GB; Class A14, AN 72-73861T XP002073469 & JP 47 043 750 B (MITSUBISHI GAS CHEMICAL I)
- DATABASE WPI Section Ch, Week 7846 Derwent Publications Ltd., London, GB; Class A14, AN 78-83115A XP002073470 & JP 53 117 094 A (MITSUBISHI GAS CHEM IND CO LTD)

## Description

### Domaine technique

La présente invention est relative à un générateur électrochimique, comprenant un matériau carboné modifié en surface par greffage de polymères.

### Art antérieur

Les matériaux carbonés jouent un rôle majeur dans de nombreux domaines, citons par exemple: les éléments de structure (matériau composite à base de fibre de carbone,...), les pigments (encre, toner, peinture,...), les électrodes (capacité à double couche, électrolyse de l'aluminium,...), les échangeurs ioniques (élimination des métaux lourds, purification de l'eau,...), les supports de catalyseurs (pile à combustible,...), les charges minérales (industrie du caoutchouc, pneu,...),...

Cette large palette d'applications est inhérente aux propriétés physico-chimiques particulières des matériaux carbonés : résistance thermique, bonne conductivité électrique, large gamme de surface spécifique (typiquement de l'ordre du m²/g au millier de m²/g), pouvoir colorant,...

Parmi tous les paramètres physico-chimiques caractérisant un matériau carboné, un des plus important est son état de surface qui conditionne au final l'interface entre le matériau carboné et le milieu environnant. Il est ainsi bien connu que différents groupements fonctionnels peuvent être présent à la surface d'un matériau carboné ou introduit postérieurement à sa synthèse par divers procédés: chimiques (oxydation, sulfonation,...), physico-chimiques (traitement électrolytique, plasma, ...) ou thermiques.

On peut ainsi introduire à la surface des matériaux carbonés diverses fonctions: radicaux, carbanions, acides carboxyliques, acides sulfoniques, acides phosphoniques, hydroxyles, amines, pyridines, halogènes, anhydrides, lactones, pyrones, quinones, etc. La concentration de ces différents groupements à la surface du carbone varie typiquement de 0,1 à 10 meq/g de carbone. Il est donc possible de contrôler et de modifier in fine les propriétés de surface de ces matériaux, par exemple leur caractère acide-base ou leur caractère hydrophile-hydrophobe. On consultera à ce sujet l'ouvrage de I.N. Ermolenko, I.P. Lyubliner & N.V. Gulko: Chemically Modified Carbon Fibers, VCH Publishers Inc., New-York, 1990. Ainsi, les poudres de carbone portant des fonctions carboxyliques à leur surface trouvent un débouché important dans le domaine des pigments pour les imprimantes à jet d'encre à haut débit (Voir Notice Technique Degussa AG: "Pigmentruße/Pigment Blacks").

Les possibilités de fonctionnalisation d'un matériau carboné sont ainsi suffisamment variées pour envisager d'initier de nombreuses réactions chimiques à partir de sa surface. Parmi toutes les opportunités de modifier les propriétés interfaciales, le greffage de polymères est d'un intérêt tout particulier. Par exemple, la présence à la surface de diverses particules ultrafines (carbone, oxyde de titane, silice, ferrite,...) d'un polymère greffé permet d'améliorer la dispersibilité des matériaux ainsi modifiés dans les solvants organiques ou les matrices polymères adéquates. On peut ainsi obtenir des dispersions colloïdales de whisker de carbone très stables dans le tétrahydrofurane en greffant à leur surface ≈ 1 g de polystyrène par gramme de carbone (N. Tsubokawa, T. Yoshihara, Y. Sone, *J. Polym. Sci., Part A, Polym. Chem.,* Vol. 30, 561-567, 1992).

Différentes stratégies de synthèse ont été mises en oeuvre pour greffer des polymères à la surface d'un matériau carboné. On peut distinguer deux grandes familles : l'initiation de la polymérisation d'un monomère à partir d'un groupement fonctionnel présent à la surface du carbone et le greffage d'un prépolymère contenant au moins une fonction chimique capable de réagir avec la surface du carbone.

Dans le premier cas, de nombreux travaux se sont attachés à initier la polymérisation de monomères par voie anionique, cationique ou radicalaire, plus souvent en modifiant un groupement fonctionnel existant sur un matériau carboné pour le rendre actif vis à vis du type de polymérisation envisagée. Citons par exemple les possibilités offertes à partir d'une fonction carboxylate facilement introduite par oxydation d'un matériau carboné : transformation en sel de métal alcalin (Li, Na, K, Cs, Rb,...) pour la polymérisation par voie anionique, transformation en sel d'acylium (perchlorate d'acylium, hexafluorure d'antimoine d'acylium,...) pour la polymérisation par voie cationique ou la transformation en peroxyesters pour la polymérisation par voie radicalaire.

Dans le second cas, d'autres travaux se sont attachés à greffer des prépolymères contenant une fonction chimique capable de réagir sur un groupement présent à la surface du carbone, la plupart du temps en modifiant le groupement présent sur le carbone pour le rendre réactif. Citons par exemple les possibilités offertes par la transformation de fonctions carboxyliques en isocyanate, en époxyde, en chlorure d'acide,... permettant ainsi de greffer par exemple un polyéthylène glycol, un alcool polyvinylique, une polyéthylène imine, un silicone diol, un silicone diamine,...

Ces différents procédés de synthèse ne sont pas complètement satisfaisant, la nécessité de modifier chimiquement le groupement fixé sur le carbone et/ou le polymère que l'on souhaite greffer rend ces synthèses complexes et coûteuses à mettre en oeuvre à grande échelle. De plus, les greffages initiés par polymérisation posent le problème des terminaisons de chaîne dont la réaction postérieure peut être défavorable au fonctionnement des systèmes intégrant ces matériaux carbonés. Ils nécessitent également de séparer les chaînes greffées sur le carbone, dès chaînes polymères qui se sont formées en solution.

### Résumé de l'invention

Afin de pallier ces limitations, les inventeurs ont étudiés ce problème et proposent, dans la présente invention, un nouveau procédé de préparation de matériaux carbonés greffés par des polymères qui consiste à attacher à la surface de matériaux carbonés des segments polymères via des liens esters ou amides par un mécanisme de déshydratation facile à mettre en oeuvre.

L'invention s'intéresse aussi à l'application de ces matériaux comme additif de conduction électronique en particulier dans les générateurs électrochimiques, comme dépôt anticorrosion et/ou conducteur électronique sur différents supports (plastique, aluminium,...) et aux encres en particulier aux encres offset.

### Description de l'invention

La présente invention propose un générateur electrochimique selon la revendication 1.

L'originalité de ce procédé réside dans le fait que la réaction est effectuée de façon à ce que le carbone que l'on souhaite modifié par des segments polymères soit en suspension dans une solution contenant le polymère que l'on souhaite greffer et dans le fait que le solvant est choisi de façon à pouvoir effectuer à une température adéquate la réaction de déshydratation entre une fonction carboxyle et une fonction amine et/ou hydroxyle.

Parmi les solvants, la diméthylformamide ou la diméthylacétamide se prêtent bien à ce type de réaction, ils permettent de dissoudre de nombreux polymères et on a pu montrer que la réaction s'effectuait en quelques heures à des températures de l'ordre de 100-150°C. Une alternative est offerte par l'utilisation de solvant permettant d'effectuer une distillation azéotropique comme le chloroforme, le tétrachlorure de carbone, le benzène, le toluène, le chlorobenzène ou le tétrachloroéthane. Ces solvants permettent d'effectuer la réaction de déshydratation en quelques heures, au maximum à la température d'ébullition du solvant, soit pour ces quatres solvants à des températures comprises entre 61 et 147°C.

Parmi les carbones fonctionnalisés, on obtient les carbones portant des fonctions amines en traitant des fibres ou des poudres de carbone par des gaz azotés (ammoniac, aminés,...) tels que décrit par exemple par Loskutov & Kuzin (*Synthesis and Properties of Ion-Exchange* Materials, (1968), Chmutov, K.V. ed., Moscow: Nauka, 95-101), cependant les carbones portant des fonctions hydroxyles ou carboxyles sont plus faciles d'accès, ils sont aisément obtenus par oxydation et sont disponibles sous formes de poudres auprès de différents fournisseurs (Cabot, Columbian Carbon, Degussa,...). Le FW 200 de la société Degussa se prête parfaitement à cette application du fait de la quantité relativement importante de groupements carboxyles à sa surface de l'ordre de 0,6 mmoles/g.

Parmi les polymères portant au moins une fonction amine, carboxyle ou hydroxyle, on préfère les polymères comportant une seule fonctionnalité capable de réagir avec la surface du carbone. Certaines familles sont particulièrement intéressantes pour cette application et en particulier les polyoxyalkylèneamines commercialisées par la société Huntsman sous le nom de Jeffamine® d'une masse molaire comprise entre 600 et 2100 g/mole (Voir notice technique Huntsman: "The Jeffamine® Polyoxyalkyleneamines"), les polyoxyalkylènes monoalcools comme le poly(éthylène glycol) méthyl éther et le poly(propylène glycol) monobutyl éther. C'est ainsi qu'on peut utiliser un polymère hydrophile choisi notamment parmi les polyoxyalkylènes monoamines.

Une classe particulière de monoamine ou de monoalcool est aussi d'un grand intérêt pratique, il s'agit non pas de polymères mais de monomères intégrant une fonction amine ou une fonction alcool et une fonction éther de vinyle (étant inclus par extension les alkyls vinyls éthers qu'on peut utiliser en tout ou en partie pour remplacer les polymères) . Comme monoamine on considère en particulier l'éther vinylique du 3-amino-1-propanol et comme monoalcool plus particulièrement l'éther vinylique de l'éthylèneglycol, l'éther vinylique du butanediol, l'éther vinylique du cyclohexanediméthanol, l'éther vinylique du diéthylèneglycol et l'éther vinylique de l'hexanediol, commercialisés par la société BASF. Le carbone ainsi modifié peut ensuite être co-réticulé avec des éthers vinyliques ou des alkyls vinyls éthers mono- ou polyfonctionnels et/ou des monomères insaturés mono- ou polyfonctionnels (maléimide, fumarate,...) permettant ainsi d'intégrer le carbone dans un réseau macromoléculaire. La réticulation avec des éthers vinyliques ou des alkyls vinyls ether peut être déclenchée par l'activation d'un amorceur de photopolymérisation sous l'effet d'un rayonnement actinique, y compris un rayonnement β. Comme photoamorceur on peut par exemple mentionner les iodoniums, les sulfoniums, les phosphoniums, les organo-métalliques permettant de libérer un acide sous l'effet d'un rayonnement actinique. On peut par exemple utiliser comme amorceur, les diaryl substitués ou non, les phénacyl-dialkyl sulfonium substitués ou non, les arènes-ferrocénium substitués ou non. La réticulation peut aussi s'effectuer en présence d'un amorceur radicalaire libérant des radicaux sous l'effet de la chaleur, phénomène bien connu de l'homme de l'art, ou encore elle peut être effectuée après avoir mis les différents composants sous forme d'un film mince, lequel peut être déposé sur un support plastique, céramique ou métallique. Par exemple, le support peut être de l'aluminium, du nickel ou du cuivre, ou une feuille de polypropylène, de polyéthylène, de polyéthylène térephtalate, de polyimide. Le greffage d'une fonction éther vinylique étant plus intéressant que de nombreux autres groupements polymérisables, une polymérisation mettant en jeu un mécanisme de type cationique présente en effet de nombreux avantages. En particulier, elle est rapide, même à basse température, le taux d'utilisation du monomère est élevé et la sensibilité aux contaminants atmosphérique, notamment l'oxygène est faible par comparaison aux polymérisations radicalaires ou anioniques.

Suivant le procédé général préféré, on fait une distillation azéotropique d'une suspension de carbone portant des fonctions carboxyles en solution dans le toluène ou dans le benzène du polymère monofonctionnel portant une fonction amine ou hydroxyle en stoechiométrie. Après 24 heures au reflux, le carbone greffé est récupéré par filtration du milieu réactionnel puis séché. Il peut être éventuellement lavé par agitation dans un solvant comme l'éther pur ou en mélange avec un co-solvant comme l'acétate d'éthyle par exemple. Lorsque le carbone greffé est soluble dans le benzène ou le toluène, on le récupére par reprécipitation du milieu réactionnel dans l'éther, filtration et séchage.

On a ainsi obtenu des particules de carbones greffés par différents segments polymères et en particulier du FW 200 greffé avec de la Jeffamine® monoaminé (polyoxyalkylènemonoamines préparés par réaction d'un alcool monohydraté avec de l'oxyde d'éthylène et/ou de propylène, suivi par la conversion du groupe hydroxyl terminol obtenu en amine) M-600 (≈ 600 g/mole), M-715, M-1000, M-2005, M-2070, avec du polyéthylène monoalcool, avec du poly(propylène glycol) monobutyl éther, avec du poly(éthylène glycol) méthyl éther, avec l'éther vinylique du 3-amino-1-propanol ou avec l'éther vinylique de l'hexanediol.

De plus, les inventeurs ont pu mettre en évidence l'intérêt de ces matériaux carbonés comme additifs de conduction électronique dans les générateurs électrochimiques. En effet, les systèmes électrochimiques doivent assurer au sein de leurs électrodes un transport simultané d'électrons et d'ions pour permettre la réalisation des réactions d'oxydo-réduction à l'origine de leur fonctionnement.

Si l'on se restreint, par souci de simplification, aux générateurs électrochimiques, il est bien connu, par exemple pour les batteries alkalines Zn/MnO₂ ou les batteries au lithium, que l'ajout d'un conducteur électronique au sein de la cathode est un élément essentiel de leur fonctionnement. Ce conducteur électronique mélangé avec le matériau électroactif et le liant pour former une électrode composite permet aux générateurs de délivrer une puissance compatible avec l'utilisation envisagée pour ces systèmes.

Le matériau carboné le plus utilisée pour cette application est le noir de carbone, tel le noir de Shawinigan, dont l'arrangement tridimensionel en chapelet assure une conductivité électronique élevée à la composite pour des taux d'addition de l'ordre de 10% en volume. Les carbones du type Ketjenblack® de la société AKZO permettent également de conférer une conductivité électronique importante à une composite par dispersion au sein de celle-ci. Contrairement au noir de Shawinigan de faible surface spécifique (≈ 10 m²/g), ces carbones présentent une surface spécifique importante de 300 à 600 m²/g. Dans ce cas, la conductivité électronique élevée induite par le Ketjenblack® est liée à la morphologie particulière des grains de carbone en "coquillage". Il est en effet couramment admis qu'une telle morphologie permet d'assurer une conduction électronique élevée par effet tunnel entre les différents grains, sans nécessité une structuration importante des grains de carbone. Citons également comme additif permettant une conductivité électronique, les graphites de surface spécifique élevée de la société Lonza.

Ces matériaux carbonés remplissent leur rôle dans les générateurs électrochimiques, les technologies utilisées dans leur fabrication permettant de réaliser des composites aptes à en assurer le fonctionnement. Malgré cela, ces matériaux présentent encore de nombreux inconvénients en terme de mise en oeuvre.

Ainsi, tous les types de carbone décrit précédemment nécessitent la mise en oeuvre de systèmes mécaniques présentant une force de cisaillement relativement élevée pour empâter les matériaux de cathode avec le carbone comme dans les batteries Zn/MnO₂ ou pour disperser de façon homogène le mélange composite matériau d'insertion, carbone et polymère dans les batteries au lithium à électrolytes polymères. De plus, il est bien connu que l'addition de carbone augmente très rapidement la viscosité des matrices polymères les contenant à des taux inférieurs à 10% en volume pour le Ketjenblack® K600 par exemple. Ceci conduit en général à l'apparition d'une porosité dans la cathode, préjudiciable à sa densité d'énergie volumique en particulier dans les batteries à électrolytes polymères. De plus, le carbone a un effet dilatant dans une matrice polymère ce qui rend l'extrusion des matrices le contenant particulièrement difficile.

On a pu montrer que la substitution des carbones utilisés habituellement dans les générateurs électrochimiques par des poudres de carbone greffées par une quantité suffisante d'un polymère adéquat était bénéfique et résolvait les problèmes exposés plus haut.

On a en particulier, évalué les performances du FW 200 greffé par de la Jeffamine M-2070 (FW-200/J-2070), composée majoritairement de polyoxyde d'éthylène. Ce matériau se présente sous l'aspect d'une poudre compacte d'aspect brillant aisément dispersable dans les solvants organiques, cet aspect étant caractéristique d'un recouvrement total de la surface du carbone par le polymère. A titre de comparaison, le FW-200 greffé par de la Jeffamine M-1000 (FW-200/J-1000) présente un aspect mat.

Dans un premier temps, on a déterminé la conductivité d'une pastille compactée de FW-200/J-2000. Il est en effet couramment admis que la modification de la surface du carbone diminue drastiquement sa conductivité. De façon suprenante, la conductivité d'une pastille compactée de FW-200/J-2000 est ≈ 2.10⁻² Ω.cm⁻¹ soit 50 S⁻¹.cm, ce qui est comparable à la conductivité observée pour de nombreuses fibres de carbone par exemple, alors que ce matériau contient en fait une fraction volumique de seulement 29% de carbone.

A l'inverse, pour une concentration volumique de 29% en volume et au vu de la surface spécifique élevée du FW-200 supérieure à 300 m²/g, il a été surprenant de constater l'aspect homogène et la bonne tenue mécanique de la pastille obtenue.

On a ainsi pu réaliser des cathodes composites de batterie au lithium à électrolytes polymères contenant un matériau d'insertion, un dérivé du polyoxyde d'éthylène et le FW-200/J-2000 ne présentant pas de porosité résiduelle contrairement à une cathode similaire utilisant le noir de Shawinigan à la même fraction volumique. On a également pu réaliser une cathode directement par compaction entre le FW-200/J-2000 et un matériau d'insertion. Cette cathode ne présentait pas de porosité résiduelle. Ces cathodes ont permis de fabriquer des batteries au lithium de performances similaires à celle utilisant du noir de Shawinigan.

Ces matériaux sont également utiles comme additif de conduction électronique dans les anodes ou les cathodes des batteries Li-Ion utilisant du carbone en lieu et place du lithium.

De plus, on a pu mettre en évidence que l'extrusion d'une matrice de polyoxyde d'éthylène contenant le FW-200/J-2000 et le sel de lithium de la bis(trifluorométhanesulfonyl)imide était beaucoup plus aisé que la même matrice contenant une fraction volumique de noir de Shawinigan.

Le FW-200/J-2000 présente aussi un intérêt pratique indéniable pour les piles Zn/MnO₂ saline. En effet, l'appauvrissement en eau de la cathode lors de la décharge est un facteur qui limite les performances de ces batteries. Or, les segments de polyoxyde d'éthylène hydrophile permettent de conserver tout au long de la décharge une hydratation suffisante de la cathode. Il en résulte un meilleur fonctionnement de ces piles primaires.

Les carbones greffés par des chaînes de polyoxyde d'éthylène sont aussi intéressants du fait de leur bonne dispersabilité et de la stabilité des suspensions colloïdales obtenues comme pigment en milieu aqueux.

Les carbones greffés par des chaînes alkyles sont particulièrement avantageux comme additifs dans les polymères peu polaires comme les caoutchoucs nitriles.

Les carbones comportant des fonctions éthers vinyliques polymérisables sont particulièrement intéressants pour effectuer des dépôts conducteurs ou pour les encres offset. On peut ainsi préparer des suspensions de carbone comportant des fonctions éthers vinyliques dans des solvants réactifs comportant des fonctions éthers vinyliques et contenant un photoamorceur. Après avoir déposé ces suspensions sur un support quelconque, on obtient des dépôts de carbone en irradiant la surface avec une lampe U.V. Ces dépôts sont particulièrement utiles pour effectuer une protection contre la corrosion des collecteurs d'aluminium utilisés dans les batteries au lithium à électrolytes liquides, gels ou polymères ; on préfère alors des solvants peu polaires limitant la diffusion des ions. D'autre part, ils permettent de réaliser des encres d'imprimerie à fixation rapide suite à une irradiation U.V. pour les procédés offset. Ils permettent également d'effectuer des dépôts conducteurs sur les anodes de lithium utilisés dans les batteries au lithium à électrolytes polymères.

Le procédé mis en oeuvre est versatile et il est également possible d'obtenir des carbones greffés avec différents polymères ou fonctions. On peut ainsi obtenir par exemple du FW-200 greffé en partie avec de la Jeffamine M-2000 et en partie avec l'éther vinylique du 3-amino-1-propanol, permettant ainsi de co-réticuler in-fine ce carbone avec un solvant réactif ou une matrice polymère adéquate.

De plus, il est bien sur évident que l'intérêt de ce procédé s'étend au greffage de polymères intégrants dans leurs chaînes des fonctionnalités quelconques tels des couples rédox, des anions délocalisés, des molécules complexantes des cations métalliques, des molécules à propriétés optiques.

Les exemples suivants servent à illustrer la mise en oeuvre de la présente invention et les applications en découlant. Ils ne présentent aucun caractère limitatif et l'homme de l'art est tout à fait capable d'imaginer des variantes sans sortir du cadre de l'invention.

L'invention est aussi illustrée sans caractère limitatif par les dessins annexés, dans lesquels:
la figure 1 illustre un générateur électrochimique incorporant un carbone greffé selon l'invention;
la figure 2 est une courbe de cyclage obtenue avec un générateur selon la figure 1;
la figure 3 illustre un générateur électrochimique incorporant un autre type de carbone greffé selon l'invention; et
la figure 4 est une courbe de cyclage obtanue avec un générateur selon la figure 3.

### Exemple 1

A une suspension dans 600 ml de toluène de 200 g d'un carbone oxydé, le FW 200 (commercialisé par la société Degussa), présentant 0,61 mmoles/g de groupements carboxyliques en surface (122 mmoles de groupements carboxyliques), on a ajouté 136 g (122 mmoles) d'un polyoxyalkylène monoaminé, la Jeffamine® M-1000 (J-1000, commercialisé par la société Huntsman), d'une masse moléculaire moyenne de 1116 g/mole. La masse moléculaire de la Jeffamine® a été déterminée par titration par l'acide chlorhydrique. Auparavant, l'eau et l'ammoniaque présent dans la Jeffamine® ont été éliminés par bullage d'argon sec à 60°C pendant 24 heures, une mesure de la quantité résiduelle d'eau par titrage Karl-Fischer inférieure à 100 ppm a confirmé la validité de ce procédé. On a alors effectué une distillation azéotropique de la suspension en maintenant une agitation à l'aide d'une olive en Teflon® aimanté. Après ≈ 30 mn, le solvant a commencé à refluer dans le Dean-Stark et l'on a observé après ≈ 10 mn l'apparition des premières gouttes d'eau. Après 20 heures au reflux, la quantité d'eau recueillie était ≈ 2,1 ml en accord avec la quantité attendue (2,2 ml pour l'amidification de 122 mmoles de -COOH) et l'on n'a plus vu apparaître de nouvelles gouttes d'eau. Le reflux a été maintenu pendant 4 heures. Après refroidissement le milieu réactionnel a été concentré à 200 ml et jeté dans 1 l d'éther. On a alors récupéré le carbone greffé par filtration, celui-ci a ensuite été séché sous vide.

Une analyse du matériau obtenu par spectroscopie infrarouge à réflexion multiple a confirmé la présence d'une bande d'absorption typique des amides et la disparition de la bande relative au groupements carboxyliques observée sur un spectre de référence du carbone oxydé non greffé. D'autre part une microanalyse du carbone greffée a donné une bonne conformité avec le résultat théorique (Tableau 1).

**Tableau 1:**

| microanalyse du FW 200 greffé par de la Jeffamine® M-1000 | | | | |
|---|---|---|---|---|
| **Carbone** | **%C** | **%H** | **%O** | **%N** |
| FW 200 greffé J-1000 | 78,09 | 4,17 | 16,05 | 0,76 |
| résultat théorique(a) | 79,20 | 3,90 | 15,30 | 0,50 |

| | | | | |
|---|---|---|---|---|
| (a) On suppose la Jeffamine® M-1000 composée de 3 motifs oxyde de propylène pour 19 motifs oxyde d'éthylène (Notice technique Huntsman: "The Jeffamine Polyoxyalkyleneamines"). | | | | |

Ce carbone greffé contient 40% en poids de polymère, il se disperse aisément dans les solvants organiques et l'eau mais l'on observe une décantation du carbone après quelques minutes lorsque l'on cesse d'agiter la dispersion.

### Exemple 2

Suivant un procédé similaire à celui décrit dans l'exemple 1, on a fait réagir 50 g de FW 200 (commercialisé par la société Degussa), présentant 0,61 mmoles/g de groupements carboxyliques en surface (30,5 mmoles de groupements carboxyliques), avec 67,7 g (30,5 mmoles) de Jeffamine® M-2070 (J-2070, commercialisé par la société Huntsman) d'une masse moléculaire moyenne de 2219 g, purifiée et caractérisée tel que décrit dans l'exemple 1. On a obtenu un matériau d'aspect brillant indiquant que la surface est enrobée de Jeffamine® M-2070.

Une analyse par spectroscopie infrarouge à réflexion multiple a confirmé le greffage de la Jeffamine® M-2070 par des liens amides. On a obtenu un résultat de microanalyse du carbone greffé conforme avec le résultat théorique escompté pour ce matériau (Tableau 2).

**Tableau 2:**

| microanalyse du FW 200 greffé par de la Jeffamine® M-2070 | | | | |
|---|---|---|---|---|
| **Carbone** | **%C** | **%H** | **%O** | **%N** |
| FW 200 greffé J-2070 | 73,10 | 4,90 | 21,30 | 0,78 |
| résultat théorique(a) | 73,40 | 5,50 | 19,50 | 0,40 |

| | | | | |
|---|---|---|---|---|
| (a) On suppose la Jeffamine® M-2070 composée de 10 motifs d'oxyde de propylène pour 32 d'oxyde d'éthylène (Notice Technique Huntsman: "The Jeffamine Polyoxyalkyleneamines). | | | | |

Ce carbone greffé contient 58% en poids de polymère, il se disperse aisément dans les solvants organiques et l'eau. Les dispersions obtenus sont stables dans le temps. On a ainsi pu obtenir une encre en milieu aqueux utile pour les stylos à encre ou à billes.

### Exemple 3

À une suspension dans 300 ml de benzène de 200 g d'un carbone oxydé, le FW 200 de la société Degussa, présentant 0,61 meq/g de groupements carboxyliques en surface (122 mmoles de groupements carboxyliques), on a ajouté 13,15 g (130 mmoles) d'un éther vinylique monoaminé, l'éther vinylique du 3-amino-1-propanol CH₂=CHO(CH₂)₃NH₂ (commercialisé par BASF). On a alors effectué une distillation azéotropique de la suspension en maintenant une agitation à l'aide d'une olive en Teflon® aimanté.

Après ≈ 30 mn, le solvant a commencé à refluer dans le Dean-Stark et l'on a observé après ≈ 10 mn l'apparition des premières gouttes d'eau. Après 12 heures au reflux, la quantité d'eau recueilli était ≈ 2,1 ml en accord avec la quantité attendue (2,2 ml pour l'amidification de 122 meq de -COOH) et l'on n'a plus vu apparaître de nouvelles gouttes d'eau. Le reflux a été maintenu pendant 2 heures. Après refroidissement, on a récupéré le carbone greffé par filtration, celui-ci a ensuite été séché sous vide.

Une analyse du matériau obtenue par spectroscopie infrarouge à réflexion multiple a confirmé la présence d'une bande d'absorption typique des amides ainsi que d'une bande d'absorption typique des éthers vinyliques et la disparition de la bande relative au groupements carboxyliques observée sur un spectre de référence du carbone oxydé non greffé.

Ce carbone greffé peut être co-réticulé avec des éthers vinyliques mono ou polyfonctionnels et/ou des monomères insaturés (maléimide, fumarate,...) permettant ainsi d'intégrer le carbone dans un réseau macromoléculaire.

### Exemple 4

Suivant un procédé similaire à celui décrit dans l'exemple 1, on a fait réagir 164 g de FW 200 (100 mmoles de groupements carboxyliques) avec 166,4 g (75 mmoles) de Jeffamine® M-2070 (J-2070) d'une masse moléculaire moyenne de 2219 g, purifiée et caractérisée tel que décrit dans l'exemple 1, et 2,53 g (25 mmoles) d'éther vinylique du 3-amino-1-propanol (commercialisé par la société BASF).

Une analyse du matériau obtenue par spectroscopie infrarouge à réflexion multiple a confirmé la présence d'une bande d'absorption typique des amides ainsi que d'une bande d'absorption typique des éthers vinyliques et la disparition de la bande relative au groupements carboxyliques observée sur un spectre de référence du carbone oxydé non greffé.

Ce matériau est particulièrement intéressant, il contient à la fois des segments polymères greffés de Jeffamine® M-2070 (50% en poids) en quantité suffisante pour assurer une dispersion aisée du carbone et obtenir des suspensions stables et des fonctions éthers vinyliques permettant de co-réticuler ce carbone avec des éthers vinyliques mono ou polyfonctionnels et/ou des monomères insaturés (maléimide, fumarate,...).

### Exemple 5

Dans un flacon en Nalgène, on a introduit 7 g (70% en poids) du carbone greffé obtenu à l'exemple 2 et 3 g (30% en poids) de poly(oxyde d'éthylène) de masse M_{w} = 3.10⁵ puis 50 ml d'acétonitrile. On a ajouté des rouleaux en zircone et le flacon a été mis en rotation sur un axe. Après 24 heures, on a obtenu une suspension stable du carbone dans la solution de poly(oxyde d'éthylène) dans l'acétonitrile. Cette suspension a alors été coulée dans un anneau en verre déposé sur une feuille d'aluminium téfloné. Pendant le temps nécessaire pour évaporer le solvant (environ 24 heures), on n'a pas observé de sédimentation du carbone greffé. Une fois le solvant évaporé, le film obtenu a été séché sous vide pendant 48 heures. Ce film qui contient en fait 29% en poids de matériau carboné (soit 20% en poids) présente de façon surprenante un aspect très homogène et une absence de porosité. Il présente une conductivité électronique supérieure à 10⁻³ Ω.cm⁻¹.

Ce résultat confirme l'intérêt des carbones greffés de l'invention pour réaliser des composites en particulier pour les électrodes de batteries au lithium à électrolytes polymères.

### Exemple 6

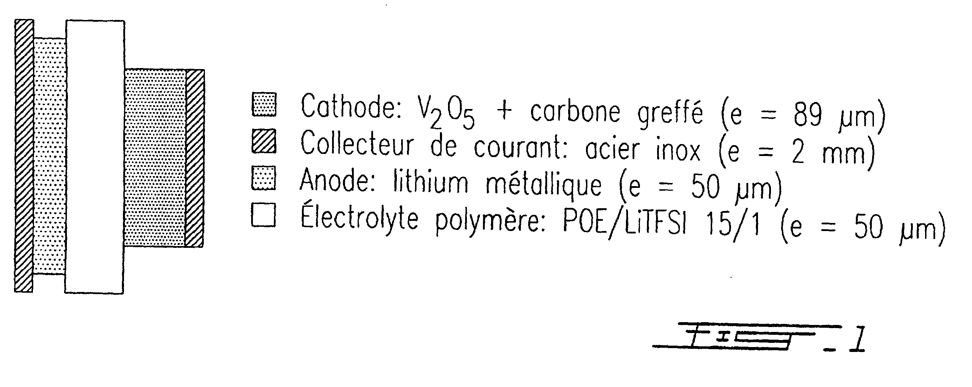

On a réalisé une cathode composite en co-broyant le carbone greffé obtenu à l'exemple 2 avec de l'oxyde de vanadium V₂O₅. On a ensuite obtenu une cathode composite compacte et non poreuse par pressage sous 2 tonnes. On a alors découpé une pastille à l'emporte-pièces et assemblé un générateur électrochimique l'utilisant comme cathode, un électrolyte polymère à base de polyoxyde d'éthylène d'une masse molaire de 5.10⁶ g/mole contenant le sel de lithium de la bis(trifluoro-méthanesulfonyl)imide à une concentration O/Li = 15/1 et une anode de lithium. On alors déposé des collecteurs de courant en acier inoxydable sur chaque face ainsi qu'on le voit sur la figure 1.

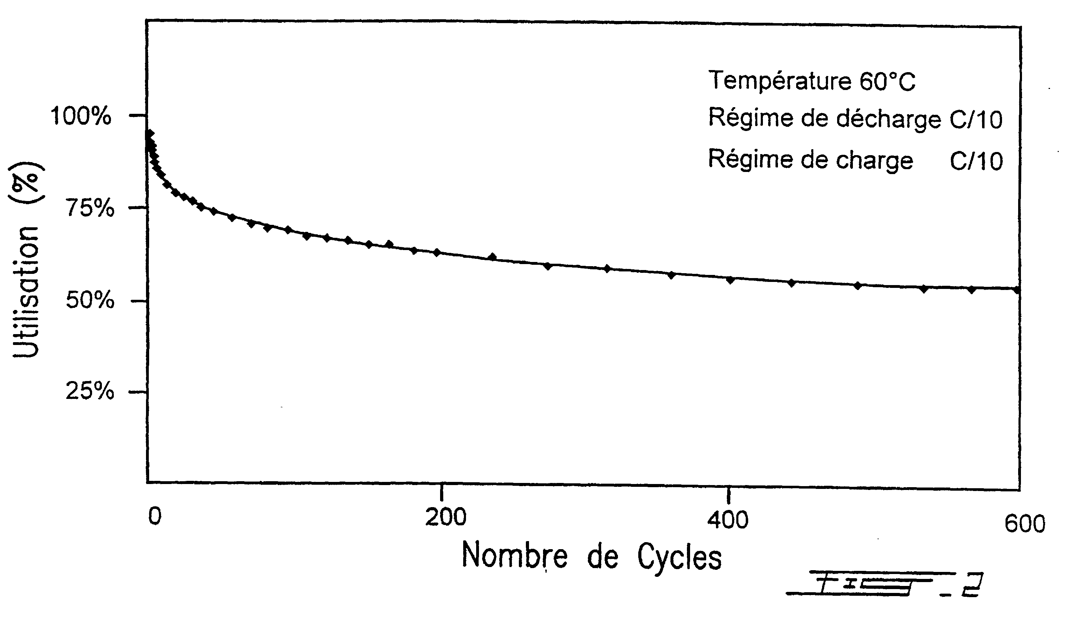

On a scellé le tout dans un boitier de pile bouton, celui-ci permettant à la fois de protéger le générateur de l'atmosphère et d'excercer une contrainte mécanique sur les films. Cette batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures). La courbe de cyclage obtenue dans ces conditions est donnée sur la figure 2.

Cette batterie a donné de très bonnes performances en cyclage, ainsi après 600 cycles, on a encore obtenu une capacité en décharge supérieure à 70% de la capacité au dixième cycle. Ce résultat confirme l'intérêt des carbones greffés de la présente invention pour les batteries au lithium à électrolytes polymères.

### Exemple 7

Dans un flacon en Nalgène, on a introduit 7 g (70% en poids) du carbone greffé obtenu à l'exemple 4, 1 g (10% en poids) du diéther vinylique du cyclohexanediméthanol (commercialisé par la société BASF) et 2 g (20% en poids) de l'éther vinylique de l'octadécyle (commercialisé par la société BASF) ainsi que 50 mg du sel de diphényliodonium de la bis(perfluorobutanesulfonyl)imide. Après avoir ajouté 20 ml d'acétonitrile, l'ensemble a été broyé par des rouleaux en zircone comme dans l'exemple 5.

Après 24 heures, la suspension obtenue a été coulée, en boite à gants sous argon, dans un anneau en verre déposé sur une feuille d'aluminium, prélablement décapée, de 20 µm d'épaisseur.
Après quatres jours en boîte à gants, le film a été irradié par un rayonnement U.V. à 254 nm, d'une puissance de 1900 mW/cm² pendant 30 secondes, suivie d'une période de 10 mn permettant la propagation des espèces générées dans le milieu (postcure). On a finalement obtenu un film d'une épaisseur de 11 µm sur la feuille d'aluminium, dénommé par la suite F1. Sur le même principe, on a réalisé un dépôt de carbone greffé (40% en poids) tel qu'obtenu à l'exemple 3 dans une matrice du diéther vinylique du cyclohexanediméthanol (60% en poids). On a ainsi obtenu un film d'une épaisseur de 8 µm sur la feuille d'aluminium, dénommé par la suite F2.

On a alors réalisé un premier générateur électrochimique utilisant une cathode composite à base de dioxyde de vanadium (45% en volume), de noir de Shawinigan (5% en volume) et de polyoxyde d'éthylène (50% en poids). On a réalisé un film à partir d'une suspension obtenue dans des conditions similaires à celle décrite dans l'exemple 5. Après séchage sous vide pendant 72 heures, on a assemblé un générateur sur le même principe que celui décrit dans l'exemple 5, mais utilisant la composite du présent exemple comme cathode et une feuille d'aluminium recouverte d'un dépôt carbone/polymère (F1) comme collecteur de courant.

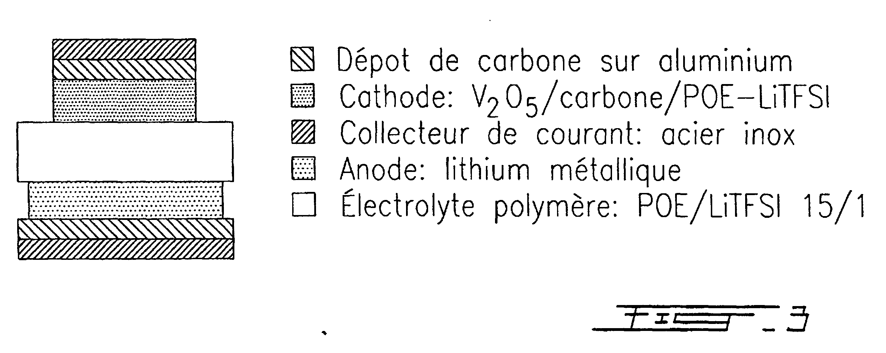

On a réalisé un second générateur électrochimique suivant le même principe mais utilisant et une feuille d'aluminium recouverte d'un dépôt carbone/polymère (F2) comme collecteur de courant. On se référera à la figure 3.

Sur le même principe, on a également réalisé à titre de comparaison un troisième générateur électrochimique utilisant une feuille d'aluminium nue prélablement décapée.

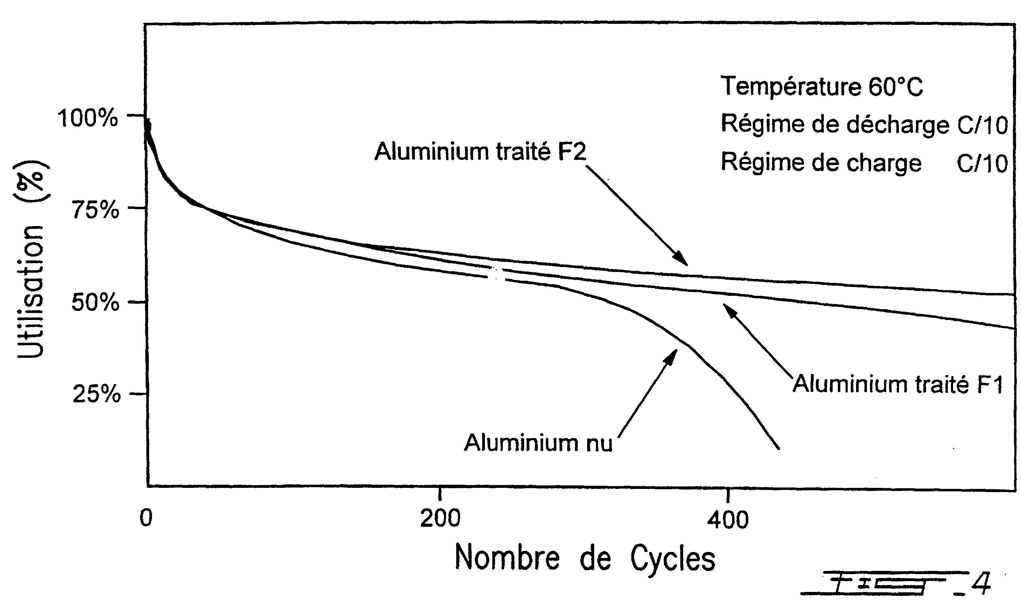

Ces trois générateurs ont été cyclés dans des conditions similaires à celle décrite dans l'exemple 6. Les courbes de cyclages obtenues dans ces conditions sont données sur la figure 4.

Comme l'illustre parfaitement cette exemple, les carbones greffés de la présente invention sont adaptés à la réalisation de dépôts anti-corrosion en particulier pour les collecteurs de courants des batteries au lithium à électrolytes polymères. Le dépôt de carbone ne contenant pas de segments polyoxyde d'éthylène est encore plus performant pour cette application, le caractère peu polaire et non solvatant de la matrice utilisé protégeant d'autant mieux de la corrosion le collecteur d'aluminium.

### Exemple 8

On a réalisé trois piles Zn/MnO₂ de technologie saline utilisant un électrolyte composé d'une solution aqueuse de chlorure d'ammonium à une concentration 1 M et une cathode composite composé de 70% en volume de dioxyde de manganèse MnO₂ et de 30% en volume de carbone greffé obtenu à l'exemple 2.

Le premier générateur a été déchargé à un régime de décharge de C/10, le second a été déchargé par intermittence à un régime de C/10 pendant 1 heure suivit d'une pause de 24 heures et ainsi de suite. Le troisième générateur a été stocké.

On a pu observer une amélioration des performances de ces piles relativement à des piles standards en particulier lors du stockage, l'autodécharge passant de 20% à 5% par semestre, et la capacité extraite de la pile étant augmenté de plus de 25% en régime intermittent.

### Exemple 9

On a réalisé une dispersion colloïdale stable de carbone greffé obtenu à l'exemple 3 (3% en poids) dans du poly-THF-divinyl éther de masse 290 (96% en poids) contenant le sel de bis(perfluorobutane-sulfonyl)imide de di(dodécylphényl)iodonium comme photoamorceur. Cette dispersion a été couché sur du papier et insolé par un rayonnement U.V. à 254 nm, d'une puissance de 1900 mW/cm² pendant quelques secondes. Le dépôt a pris quasi-instantanément en masse. On a également obtenu de bons résultat en diluant cet encre par de l'eau.

De telles compositions d'encres sont particulièrement intéressant pour l'imprimerie offset, de par l'absence de solvant organique dans le procédé. De plus la vitesse de polymérisation est compatible

### Exemple 10

On a réalisé une solution dans la dicholorométhane d'un caoutchouc nitrile (96%) en poids) en présence d'un carbone similaire à celui greffé avec du polyéthylène de masse 800, obtenu par un procédé similaire à celui décrit dans l'exemple 1 en remplaçant la Jeffamine® monoaminé par un polyéthylène monoalcool de masse 800 (commercialisé par Aldrich). Le carbone greffé se disperse aisément dans ce solvant. Après coulage de la solution visqueuse et séchage, on a obtenu une dispersion homogène de carbone dans le polymère.

Ce polymère a une conductivité de 10⁻⁶ S.cm.⁻¹ et des propriétés antistatiques.

Il est entendu que des modifications sont possibles sans sortir du cadre de la présente invention telle que définie dans les revendications qui suivent.

## Revendications

1. Générateur électrochimique **conforme à la technologie Li-polymère ou Li-Ion,** constitué d'une anode, d'une cathode et d'un électrolyte, dans lequel l'anode et/ou la cathode comporte un matériau carboné modifié en surface, obtenu par un procédé consistant à :
- mettre un matériau carboné comportant des fonctions carboxyles à sa surface, en suspension dans une solution comprenant au moins un polymère et/ou au moins un monomère réticulable à greffer sur ledit matériau carboné, ladite solution comprenant aussi un solvant dudit polymère ou monomère, puis
- traiter le mélange réactionnel dans des conditions favorisant une réaction de déshydratation entre une fonction carboxyle et une fonction amine et/ou hydroxyle, de façon à greffer au moins un desdits polymères sur le matériau carboné par des liens esters ou amides ;
- lorsque le milieu réactionnel contient un monomère réticulable, réticuler ledit monomère,
**caractérisé en ce que** ledit polymère est choisi parmi les polyoxyalkylènes monoamines et les polyoxyalkylènes monoalcools, et **en ce que** le monomère est un monomère comportant une fonction amine ou une fonction alcool et une fonction éther de vinyle ou alkyl vinyl éther.

2. Générateur électrochimique selon la revendication 1, **caractérisé en ce que** ledit polymère est choisi parmi les polyoxyalkylènemonoamines préparés par réaction d'un alcool monohydraté avec de l'oxyde d'éthylène et/ou de propylène, suivi par la conversion du groupe hydroxyl terminol obtenu en amine, le poly(éthylène glycol) méthyl éther, et le poly(éthylène glycol) monobutyléther.

3. Générateur électrochimique selon la revendication 1, **caractérisé en ce que**, dans la première étape du procédé, on utilise un monomère comportant une fonction amine ou une fonction alcool et une fonction éther de vinyle ou alkyl vinyl éther, en l'absence de polymère.

4. Générateur électrochimique selon la revendication 1, **caractérisé en ce que**, dans la première étape du procédé, on utilise un.polymère.comportant une fonction amine ou une fonction alcool, en l'absence de monomère.

5. Générateur électrochimique selon la revendication 1, **caractérisé en ce que**, dans la première étape du procédé, on utilise un polymère et un monomère comportant une fonction amine ou une fonction alcool et une -fonction éther de vinyle ou alkyl vinyl éther.

6. Générateur électrochimique selon les revendications 3 ou 5, **caractérisé en ce que** le monomère est choisi parmi l'éther vinylique du 3-amino-1-propanol, l'éther vinylique de l'éthylène glycol, l'éther vinylique du butanediol, l'éther vinylique du cyclohexanediméthanol, l'éther vinylique du diéthylèneglycol et l'éther vinylique de l'hexanediol.

7. Générateur électrochimique selon les revendications 3 ou 5, **caractérisé en ce que** la carbone modifié est réticulé avec des éthers vinyliques ou des alkyl vinyl éthers mono- ou polyfonctionnels.

8. Générateur électrochimique selon les revendications 3 ou 5, **caractérisé en ce que** le carbone modifié est réticulé avec des éthers vinyliques ou des alkyl vinyl éthers mono- ou polyfonctionnels et des monomères insaturés mono- ou polyfonctionnels.

9. Générateur électrochimique selon la revendication 7 ou 8, **caractérisé en ce que** la réticulation est effectuée après avoir mis les différents composants sous forme d'un film mince.

10. Générateur électrochimique selon la revendication 9, **caractérisé en ce que** le film mince est déposé sur un support plastique, céramique ou métallique.

11. Générateur électrochimique selon la revendication 10, **caractérisé en ce que** le support est de l'aluminium, u nickel, du zinc, du lithium ou du cuivre.

12. Générateur électrochimique selon la revendication 1, **caractérisé en ce que** le matériau carboné est du noir de carbone.

13. Générateur électrochimique selon la revendication 1, **caractérisé en ce que** le polymère à greffer comporte dans sa chaîne d'autres fonctions choisies parmi les couples rédox, les anions délocalisés, les molécules complexantes de cations métalliques ou des molécules à propriétés optiques.

14. Générateur selon la revendication 11, **caractérisé en ce que** le film mince déposé sur un substrat constitue un collecteur de courant.

## Claims

1. An electrochemical generator of the Li-polymer or the Li-ion technology, consisting of an anode, a cathode and an electrolyte, wherein the anode and/or the cathode comprises a carbonated material modified at its surface, obtained by a method consisting in :
- suspending a carbonated material containing carboxyl functions at its surface, in a solution comprising at least one polymer and/or at least one cross-linkable monomer to be grafted on the said carbonated material, the said solution furthermore containing a solvent of said polymer or monomer, then
- treating the reaction mixture under conditions promoting a reaction of dehydration between a carboxyl function and an amine and/or hydroxyl function, so as to graft at least one of the said polymers on the carbonated material by means of ester or amide bonds ;
- when the reaction mixture contains a monomer, cross-linking said monomer ;
**characterized in that** the said polymer is selected from polyoxyalkylene monoamines and polyoxyalkylene monoalcohols, and **in that** the monomer is a monomer containing an amine function or an alcohol function and a vinyl ether function or an alkyl vinyl ether function.

2. An electrochemical generator according to claim 1, **characterized in that** the said polymer is selected from polyoxyalkylene monamines prepared by reacting a monohydrated alcohol with ethylene or propylene oxide and converting the obtained hydroxyl end group into an amine, poly(ethylene glycol) methyl ether, and poly (ethylene glycol) monobutyl ether.

3. An electrochemical generator according to claim 1, **characterized in that**, in the first step of the method, a monomer having an amine function or an alcohol function and a vinyl ether function or an alkyl vinyl ether function is used without a polymer.

4. An electrochemical generator according to claim 1, **characterized in that**, in the first step of the method, a polymer having an amine function or an alcohol function is used, without a monomer.

5. An electrochemical generator according to claim 1, **characterized in that**, in the first step of the method, a polymer and a monomer having an amine function or an alcohol function and a vinyl ether function or an alkyl vinyl ether function are used.

6. An electrochemical generator according to claims 3 or 5, **characterized in that** the monomer is selected from 3-amino-1-propanol vinyl ether, ethylene glycol vinyl ether, butanediol vinyl ether, cyclohexanedimethanol vinyl ether, diethylene glycol vinyl ether and hexanediol vinyl ether.

7. An electrochemical generator according to claims 3 or 5, **characterized in that** the modified carbon is cross-linked with mono- or polyfunctional vinyl ethers or alkyl vinyl ethers.

8. An electrochemical generator according to claims 3 or 5, **characterized in that** the modified carbon is cross-linked with mono- or polyfunctional vinyl ethers or alkyl vinyl ethers and mono- or polyfunctional unsaturated monomers.

9. An electrochemical generator according to claims 7 or 8, **characterized in that** the cross-linking is carried out after having provided the various components in the form of a thin film.

10. An electrochemical generator according to claim 9, **characterized in that** the thin film is placed on a plastic, ceramic or metallic support.

11. An electrochemical generator according to claim 10, **characterized in that** the support comprises aluminium, nickel, zinc, lithium or copper.

12. An electrochemical generator according to claim 1, **characterized in that** the carbonated material is carbon black.

13. An electrochemical generator according to claim 1, **characterized in that** the polymer to be grafted integrates in its chain other functions selected from redox couples, delocalised anions, metallic cations complexing molecules and molecules with optical properties.

14. An electrochemical generator according to claim 11, **characterized in that** the thin film placed on a support forms a current collector.

## Patentansprüche

1. Elektrochemische Li-Polymer- oder Li-Ionen-Zelle, die aus einer Anode, einer Kathode und einem Elektrolyten besteht, worin die Anode und/oder die Kathode ein oberflächenmodifiziertes kohlenstoffhältiges Material umfasst, erhalten durch ein Verfahren, das aus Folgendem besteht:
- Suspendieren eines Carboxylgruppen-hältigen kohlenstoffhältigen Materials in einer Lösung, die zumindest ein Polymer und/oder zumindest ein vernetzbares Monomer zum Aufpfropfen auf das kohlenstoffhältige Material enthält, wobei die Lösung auch ein Lösungsmittel des Polymers oder Monomers umfasst, dann
- Behandeln des Reaktionsgemisches unter Bedingungen, die eine Dehydratationsreaktion zwischen einer Carboxylfunktionalität und einer Amin- und/oder Hydroxylfunktionalität begünstigen, so dass zumindest eines des Polymere durch Ester- oder Amidbindungen auf das kohlenstoffhältige Material aufgepfropft wird;
- wenn das Reaktionsmedium ein vernetzbares Monomer enthält, Vernetzen des Monomers,
**dadurch gekennzeichnet, dass** das Polymer aus Polyoxyalkylenmonoaminen und Polyoxyalkylenmonoalkoholen ausgewählt ist und dass das Monomer ein Monomer ist, das eine Aminfunktionalität oder eine Alkoholfunktionalität und eine Vinylether- oder Alkylvinyletherfunktionalität umfasst.

2. Elektrochemische Zelle nach Anspruch 1, **dadurch gekennzeichnet, dass** das Polymer aus Polyoxyalkylenmonoaminen, die durch Umsetzung eines einwertigen Alkohols mit Ethylen- und/oder Propylenoxid, gefolgt von der Umwandlung der erhaltenen endständigen Hydroxylgruppe in ein Amin hergestellt sind, Poly(ethylenglykol)methylether und Poly(ethylenglykol)monobutylether ausgewählt ist.

3. Elektrochemische Zelle nach Anspruch 1, **dadurch gekennzeichnet, dass** im ersten Schritt des Verfahrens ein Monomer, das eine Aminfunktionalität oder eine Alkoholfunktionalität und eine Vinylether- oder Alkylvinyletherfunktionalität umfasst, ohne Polymer eingesetzt wird.

4. Elektrochemische Zelle nach Anspruch 1, **dadurch gekennzeichnet, dass** im ersten Schritt des Verfahrens ein Polymer, das eine Aminfunktionalität oder eine Alkoholfunktionalität umfasst, ohne Monomer eingesetzt wird.

5. Elektrochemische Zelle nach Anspruch 1, **dadurch gekennzeichnet, dass** im ersten Schritt des Verfahrens ein Polymer und ein Monomer mit einer Aminfunktionalität oder einer Alkoholfunktionalität und einer Vinylether- oder Alkylvinyletherfunktionalität eingesetzt werden.

6. Elektrochemische Zelle nach einem der Ansprüche 3 oder 5, **dadurch gekennzeichnet, dass** das Monomer aus 3-Amino-1-propanolvinylether, Ethylenglykolvinylether, Butandiolvinylether, Cyclohexandimethanolvinylether, Diethylenglykolvinylether und Hexandiolvinylether ausgewählt ist.

7. Elektrochemische Zelle nach einem der Ansprüche 3 oder 5, **dadurch gekennzeichnet, dass** der modifizierte Kohlenstoff mit mono- oder polyfunktionellen Vinylethern oder Alkylvinylethern vernetzt ist.

8. Elektrochemische Zelle nach einem der Ansprüche 3 oder 5, **dadurch gekennzeichnet, dass** der modifizierte Kohlenstoff mit mono- oder polyfunktionellen Vinylethern oder Alkylvinylethern und ungesättigten mono- oder polyfunktionellen Monomeren modifiziert ist.

9. Elektrochemische Zelle nach Anspruch 7 oder 8, **dadurch gekennzeichnet, dass** die Vernetzung durchgeführt wird, nachdem die verschiedenen Bestandteile in die Form eines dünnen Films gebracht worden sind.

10. Elektrochemische Zelle nach Anspruch 9, **dadurch gekennzeichnet, dass** der dünne Film auf einen Träger aus Kunststoff, Keramik oder Metall aufgetragen ist.

11. Elektrochemische Zelle nach Anspruch 10, **dadurch gekennzeichnet, dass** der Träger aus Aluminium, Nickel, Zink, Lithium oder Kupfer besteht.

12. Elektrochemische Zelle nach Anspruch 1, **dadurch gekennzeichnet, dass** das kohlenstoffhältige Material Ruß ist.

13. Elektrochemische Zelle nach Anspruch 1, **dadurch gekennzeichnet, dass** das zu pfropfende Polymer in seiner Kette andere Funktionalitäten aufweist, die aus Redoxpaaren, delokalisierten Anionen, Metallkationen komplexierenden Molekülen oder Molekülen mit optischen Eigenschaften ausgewählt sind.

14. Zelle nach Anspruch 11, **dadurch gekennzeichnet, dass** der auf ein Substrat aufgetragene dünne Film einen Stromkollektor bildet.
